# EUROPEAN PATENT SPECIFICATION

(11) **EP 1 243 914 B1**
(45) Date of publication and mention of the grant of the patent: **08.12.2004**
(21) Application number: 02006567.8
(22) Date of filing: 21.03.2002
(51) Int. Cl.: G01N 3/08, G01N 33/483

(54) **Method of evaluating degree of hair damage**
Verfahren zur Abschätzung von Haarschäden
Procédé de l'estimation du dommage des cheveux

(30) Priority: 23.03.2001 JP 2001086115
(43) Date of publication of application: 25.09.2002
(73) Proprietor: KAO CORPORATION, Chuo-ku Tokyo (JP)
(72) Inventor: Oishi, Susumu, Kao Corporation, Tokyo (JP); Yamada, Shinji, Kao Corporation, Ichikai-machi, Haga-gun, Tochigi (JP); Fujikura, Yoshiaki, Kao Corporation, Ichikai-machi, Haga-gun, Tochigi (JP)
(74) Representative: HOFFMANN - EITLE

(56) References cited:
- EP-A- 0 388 247
- GB-A- 2 104 225
- US-A- 3 324 713
- PATENT ABSTRACTS OF JAPAN vol. 011, no. 062 (P-551), 25 February 1987 (1987-02-25) & JP 61 226632 A (KANEBO LTD), 8 October 1986 (1986-10-08)
- J. ALAN SWIFT: "The mechanics of fracture of human hair" INTERNATIONAL JOURNAL OF COSMETIC SCIENCE, vol. 21, 1999, pages 227-239, XP002257016

## Description

### Field of Invention:

The present invention relates to a method of evaluating the degree of hair damage based on the tear strength of a hair fiber in its longitudinal direction.

### Description of the Related Art:

Human hair can be damaged due to various factors : (i) daily hair care such as shampooing, blowing, and brushing; (ii) chemical treatments with permanent wave solutions, bleaching agents, hair dyes, etc.; and (iii) environment factors such as ultraviolet light and spontaneous oxidation. Known techniques for evaluating the degree of hair damage include, for example, the method disclosed in JP-B-3-10899.

According to this method, a hair fiber is stretched in longitudinal direction and the elastic limit is obtained from the stress-strain curve. The degree of damage is evaluated by comparing the elastic limit of the hair fiber sample with that of reference sample.

### SUMMARY OF THE INVENTION

The hair damage evaluation method based on the stretching properties described above is effective for heavily damaged hair; the elastic limit of such hair fiber is very different from that of reference sample. However, it is difficult to evaluate the degree of hair damage using this method where the damage is in an initial stage or the degree of damage is light, because elastic limit of such hair fiber is not very different from reference value (tensile strength is not a sensitive index for evaluating the degree of hair damage).

An object of the present invention is to provide a method of evaluating the degree of hair damage even when the hair damage is in an initial stage or light.

The present inventors have found that the strength of a hair fiber tearing in its longitudinal direction, i.e., splitting is a more sensitive damage index than the conventional tensile strength.

Completed based on the above finding, the present invention provides a method of evaluating the degree of hair damage which comprises tearing a hair fiber in its longitudinal direction to obtain a tear strength and evaluating the degree of damage based on the tear strength.

According to the present invention, the degree of hair damage can be evaluated even when the damage is in its initial stage or light.

### BRIEF DESCRIPTION OF THE DRAWINGS

Fig.1 is the schematic illustration of a device that can be used to prepare a hair fiber sample.
Fig.2 shows the relationship between tearing force and time; tear strength, the damage index in this invention, is obtained from this measurement
Fig.3 represents the results of damage evaluation carried out in Example and Comparative Example, in which Fig.3A shows the results of tear strength measurements, and Fig. 3B shows the results of tensile strength measurements.

### DETAILED DESCRIPTION OF THE INVENTION

The present invention will be described below based on its preferred embodiments with reference to the accompanying drawings.

According to the damage evaluation method of the present invention, a hair fiber is torn apart in its longitudinal direction to obtain tear strength, and the degree of hair damage is evaluated based on the tear strength.

The tear strength of a hair fiber is obtained from a tearing force needed to tear apart a hair fiber into symmetrical halves.

The tearing force is obtained as follows. One end of a hair fiber sample is split into two with a knife, etc. to initiate a tear, and the sample is clamped by jaws at both split ends. One or both of the jaws are relatively moved in a straight line in order to tear apart the fiber in the longitudinal direction. The force needed to tear is measured with a load meter equipped with a load cell (e.g., a commercially available force gauge).

The length of the initial tear made in a hair fiber by knife is preferably 0.5 to 5 mm, still preferably 1 to 3 mm, for securing ease of clamping and symmetry of splitting (the hair should be split in substantially equal halves).

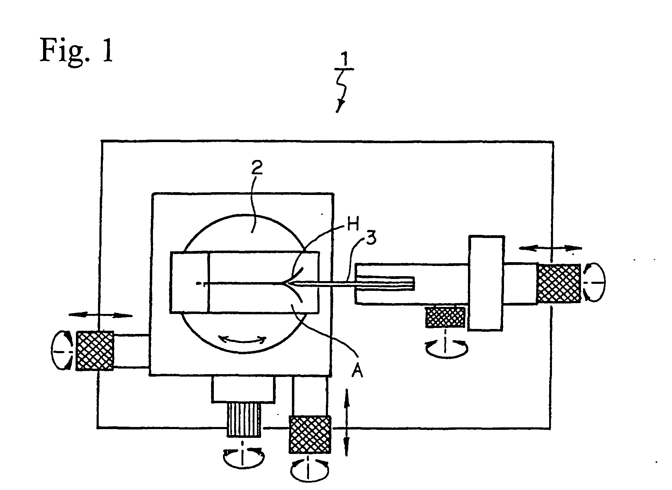

The initial tear (or split) in one end of a hair fiber sample can be made by means of, for example, a cutting device shown in Fig.1. The cutting device 1 of Fig.1 has a stage 2 on which a hair fiber sample H is fixed and a cutter 3 used to split one end of the hair fiber sample H to a predetermined length.

The stage 2 is designed to move in two crossing direction and revolve on its own vertical axis in a horizontal plane so that the hair fiber sample H fixed on an aluminum plate A may be positioned accurately.

The cutter 3 is designed to move back and forth in a horizontal plane and to move up and down in the vertical direction so that it may be positioned accurately in agreement with the fixed position of the hair fiber H.

One end of the hair fiber H fixed on the stage 2 is split into two in a prescribed length with the cutter 3 under observation through an optical microscope. It is important that the initial split be made through the center of the hair fiber to divide into symmetrical halves.

In measuring the tearing force, the hair fiber sample is torn apart over a predetermined length. The tearing length after initial tear is preferably 0.5 to 30 mm, still preferably 1 to 10 mm, for securing ease of handling and for preventing deviation of the split direction from the center (which results in asymmetry).

For tearing the hair fiber, it is preferred to pull one of the split ends at a constant speed with the other split end fixed. This mode is suitable to tear apart the hair fiber into equal halves along with its longitudinal direction. Further, this mode is fit for measuring in water as described later.

The tearing (pulling) speed is preferably 0.2 to 10 mm/min, still preferably 0.5 to 5 mm/min, for facilitating tearing apart the hair fiber into substantially equal halves and obtaining tear strength data stably.

It is preferred that the atmosphere in which the hair fiber is torn after an initial split should be controlled at substantially constant temperature and humidity so as to minimize the influences of environmental conditions. In particular, damage evaluation of heavily damaged hair, such as hair damaged from chemical treatments, is preferably carried out in water, such as pure water or ion-exchanged water, at a prescribed temperature.

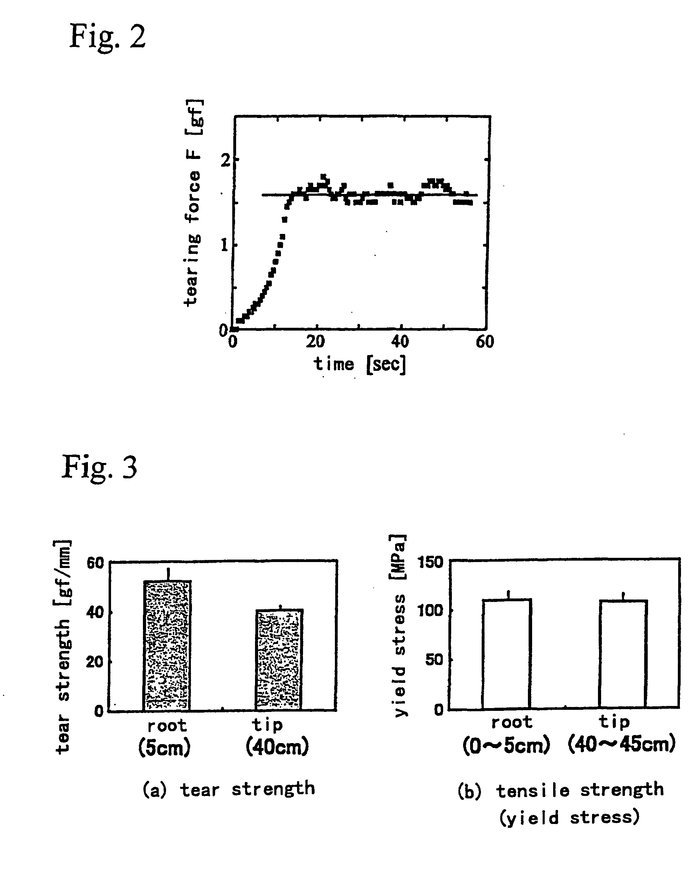

Fig. 2 is a graph showing an example of the result of tearing force measurement. In the graph tearing force (gf) is plotted as ordinate and time (sec) as abscissa.

As shown in Fig. 2, the force steeply rises with time and then reaches a plateau. The initial steep rise is attributed to the stretching of the sag in the initially split part of the hair fiber. An actual tear occurs in the plateau region (from about 13 seconds after in Fig.2). The plateau force is taken as a tearing force, and the tear strength of the sample is obtained by dividing the tearing force by the diameter of the hair fiber. For enhancing accuracy, the tearing force to be used for calculation is obtained through conventional statistical processing.

The hair damage evaluation according to the present invention is preferably achieved by comparing the measured tear strength with a reference tear strength. In order to exclude individual differences and the factor of damage caused by daily hair care, the reference tear strength is preferably a tear strength obtained in the same manner as described above using a hair fiber sample cut near the root from the same person. A tear strength of a healthy hair that has undergone no chemical treatments with permanent wave solutions, bleaching agents, etc. may also be used as a reference sample. The term "near the root" as used herein indicates the part 0 to 5 cm distant from the hair root. Various parameters such as the ratio of the tear strength between evaluation sample and reference sample, the difference between them, can be used as the damage evaluation indexes. The degree of hair damage can be graded using these indexes.

The hair damage evaluation method of the present invention is useful to evaluate not only the degree of hair damage but also the degree of the recovery of hair damage. That is, the method is advantageously applicable to research and development of commodities such as personal hair care products, e.g., shampoos, rinses, and conditioners, and hair chemicals, e.g., permanent wave solutions, bleaching agents, and hair dyes; the degree of recovery of hair damage using these hair care products can be evaluated by this method.

The hair damage evaluation method of the present invention is particularly suited for human hair of the head. As a matter of course, it is also applicable to evaluating the hairs of eyebrows, eyelashes, a mustache or a beard and animal hairs.

In Example hereinafter given, hair damage evaluation was carried out using the tear strength as the damage index; the results were compared with the damage evaluation based on the tensile strength measurements. Fig. 3 shows an average tear strength (n=4) and an average tensile strength (n=3) with their standard deviation (error bars). Fig. 3 also shows a reference tear strength, which was obtained from a hair fiber cut from the position 5 cm distant from the hair root, and a reference tensile strength, obtained from a hair fiber cut from the part 0 and 5 cm distant from the hair root.

### Hair for evaluation:

Sample hair fibers were collected from a 27-year-old female wearing about 45 cm-long straight hair having received no chemical treatments such as permanent wave and dye. The hair showed no particular damage as far as their luster and texture.

### EXAMPLE

### Preparation of hair fiber samples:

Almost 2 cm-length was cut out from each of four hair fiber samples between 38 cm and 40 cm from the root. Near tip end was split into substantially equal halves to a length of about 2 mm using the cutting device shown in Fig. 1 under observation through an optical microscope.

### Measurement of tear strength:

Both split ends were clamped with jaws; one was fixed and the other was movable. The movable jaw was pulled up at a constant speed (2 mm/min) and the hair fiber was torn apart into halves. Tearing force (gf) was measured with a digital force gauge (AD-4937, A&D Corp. Japan). As shown in Fig. 2, the force was plotted against times and tearing force (plateau force) was measured; tear strength (gf/mm) was obtained by dividing the tearing force by the diameter of hair fiber.

### COMPARATIVE EXAMPLE

### Preparation of hair fiber samples:

Almost 5 cm-length was cut out from each of three hair fibers between 40 cm and 45 cm distant from the hair root.

### Measurement of tensile strength:

Tensile strength of the hair fiber was measured using a tensile tester (Solid Analyzer RSA II of Rheometrics). A stress-strain curve was obtained by pulling the hair fiber at a constant rate (10 mm/min) in an atmosphere of 24 °C and 40% RH. The yield stress in the curve was taken as a tensile strength.

As shown in Fig. 3A, the tear strength of the hair fiber sample (near the tip) was 25% smaller than that of the reference strength (obtained from the hair sample near the root). On the other hand, the stress-strain curve of the hair fiber cut from near the tip overlap with that of the hair fiber cut from near the root, giving no significant difference in the tensile strength (yield stresses) between root and tip.

It has thus been ascertained that the tear strength measurement described in this invention enables quantitative evaluation of hair damage with high sensitivity, even where the damage is in its initial stage before reaching a level detectable by the feel or the conventional tensile strength measurements.

## Claims

1. A method of evaluating the degree of hair damage carachterised in that it comprises tearing a hair fiber in its longitudinal direction to obtain a tear strength and evaluating the degree of damage based on said tear strength.

2. A method of evaluating the degree of hair damage according to claim 1, wherein the degree of damage is evaluated by comparing said tear strength with reference tear strength.

## Patentansprüche

1. Verfahren zur Bewertung des Grades der Haarschädigung, **dadurch gekennzeichnet, dass** es das Ziehen einer Haarfaser in Längsrichtung, wobei eine Zugfestigkeit erhalten wird, und Bewertung des Schädigungsgrades auf Grundlage der Zugfestigkeit umfasst.

2. Verfahren zur Bewertung des Grades der Haarschädigung gemäss Anspruch 1, wobei der Grad der Schädigung durch Vergleich der Zugfestigkeit mit einer Referenzzugfestigkeit bewertet wird.

## Revendications

1. Procédé pour évaluer le degré d'endommagement des cheveux **caractérisé en ce qu'**il comprend de déchirer une fibre capillaire dans sa direction longitudinale pour obtenir une résistance au déchirement et d'évaluer le degré d'endommagement sur la base de ladite résistance au déchirement.

2. Procédé pour évaluer le degré d'endommagement des cheveux selon la revendication 1, dans lequel le degré d'endommagement est évalué en comparant ladite résistance au déchirement avec une résistance au déchirement de référence.
